# EUROPÄISCHE PATENTSCHRIFT

(11) **EP 0 667 337 B1**
(45) Veröffentlichungstag und Bekanntmachung des Hinweises auf die Patenterteilung: **21.01.1998**
(21) Anmeldenummer: 95101052.9
(22) Anmeldetag: 26.01.1995
(51) Int. Cl.: C07C 211/52, C07C 209/36

(54) **Verfahren zur Herstellung von Fluoranilinen**
Method for preparing fluoroanilines
Procédé pour la préparation de fluoranilines

(30) Priorität: 11.02.1994 DE 4404342; 16.12.1994 DE 4444903
(43) Veröffentlichungstag der Anmeldung: 16.08.1995
(73) Patentinhaber: Clariant GmbH, 65929 Frankfurt am Main (DE)
(72) Erfinder: Schach, Thomas, Dr., D-64579 Gernsheim (DE); Papenfuhs, Theodor, Dr., D-60433 Frankfurt am Main (DE)

(56) Entgegenhaltungen:
- EP-A- 0 001 825
- EP-A- 0 506 199
- EP-A- 0 562 435
- US-A- 4 140 719
- US-A- 4 294 988
- US-A- 5 041 674

## Beschreibung

Die vorliegende Erfindung betrifft ein verbessertes Verfahren zur Herstellung von Fluoranilinen durch Reduktion der Nitrogruppe und katalytische Halogenabspaltung brom- und chlorhaltiger Fluornitrobenzole.

Fluoraniline finden eine breite Anwendung im Bereich des Pflanzenschutzes und als Synthesebausteine in Pharmavorprodukten.

Durch elektrophile und nukleophile Substitution können Aromaten abhängig von ihrem vorhandenen Substitutionsmuster nur an ganz bestimmten Positionen weiter substituiert werden. In der Synthese von Aromaten tritt aber oft der Umstand ein, daß gerade an den weniger bevorzugten Positionen ein Substituent eingebracht werden muß. Es existieren nun eine Reihe von Strategien, dieses Problem zu lösen. So lassen sich unerwünschte Positionen im Aromaten mit Substituenten blockieren, die einerseits einfach ins Molekül einzuführen sind und andererseits ebenso einfach wieder entfernt werden können. Als Substituenten der Wahl bieten sich die Halogene Brom und Chlor an, die sehr einfach durch elektrophile Substitution in ein aromatisches System eingeführt werden, diese Position im Molekül für weitere Angriffe sperren, gegebenenfalls die elektronischen Verhältnisse im Molekül für den Eintritt weiterer Substituenten günstig beeinflussen und deren Abspaltung am Ende der Synthesesequenz einfach möglich ist.

Fluornitrobenzole lassen sich zum Teil auf elegante Weise durch Halexreaktionen aus den entsprechenden Chlornitrobenzolen synthetisieren. Bestimmte Substitutionsmuster sind dabei nur auf Umwegen zugänglich da die entsprechende Ausgangsverbindungen nur äußerst schwierig zu synthetisieren sind. Hochsubstituierte Chlornitroverbindungen, die neben nicht benötigten Chloratomen, das gewünschte Chlornitrostrukturinkrement beinhalten, lassen sich hingegen auf einfache und technisch günstige Weise herstellen. Nach der Substitution der reaktiven Chlorpositionen durch Fluor, können die verbleibenden Chloratome am Ende der Synthese unter gleichzeitiger Reduktion der Nitrogruppe wieder entfernt werden . Dafür bietet sich die reduktive Enthalogenierung an.

Neben diesen exemplarischen Beispielen sind noch eine Vielzahl von Möglichkeiten denkbar, in denen die reduktive Halogenabspaltung eingesetzt werden kann. Beispielsweise können in Austauschreaktionen isomere Chlornitroverbindungen verwendet werden, wenn nach dem Austausch die entstandenen Chlorfluornitroverbindungen das Strukturinkrement des gewünschten Fluoranilins beinhalten, liefert die anschließende reduktive Enthalogenierung mit Reduktion der Nitrogruppe das isomerenreine Endprodukt.

Allerdings treten bei der Durchführung dieser Reaktion eine Reihe verfahrenstechnischer Probleme auf, die bislang nicht zufriedenstellend gelöst werden konnten.

Die Reaktionen werden im allgemeinen in Gegenwart eines Katalysators wie beispielsweise Palladium, eines Lösungsmittels und einer wässrigen Base wie beispielsweise Natronlauge durchgeführt. Reduktive Enthalogenierungen von Chlor-/Brom-Fluornitrobenzolen liefern unter diesen Reaktionsbedingungen nur mäßige Selektivitäten und Ausbeuten (US-A-4294988 und EP-A-562435). In der Regel verlaufen diese Reaktionen nur schwer reproduzierbar (Katalysator-Vergiftungen) und die Selektivität wird durch die Abspaltung von Fluor deutlich verschlechtert. Chlorid-Korrosion ist in vielen Fällen nicht zu vermeiden, da für die meisten der Enthalogenierungen Reaktionstemperaturen von 100° bis 150° C benötigt werden und in dipolar protischen Lösungsmitteln gearbeitet wird.

Für den Fall der Fluoridabspaltung treten zwei in vielen Fällen nicht zu lösende Probleme in den Vordergrund. Zum einen lassen sich die mit unterschiedlichen Fluoridgehalten gebildeten Rohprodukte praktisch nicht oder nur mit einem sehr aufwendigen Trennverfahren reinigen, da ihre Siedepunkte in den meisten Fällen praktisch identisch sind. Zum anderen kann das in der Reaktion gebildete Fluorid zu weiterer Korrosion führen, der nur mit hohen Ansprüchen an das Reaktormaterial begegnet werden kann.

Desweiteren führt die hohe Nukleophilie der verwendeten Base (bspw.: wässrige NaOH) oder des Lösungsmittels (Wasser; Alkohole in Kombination mit den verwendeten Basen) unter den Reaktionsbedingungen zur Bildung von Nebenprodukten, wodurch die Selektivität dieser Reaktion weiter verschlechtert wird. Werden die üblichen Amine wie beispielsweise Trimethylamin, Triethylamin verwendet (EP-A-001825), kann die Bildung von Nebenprodukten weitestgehend unterdrückt werden. Die gebildeten Salze dieser Basen, bzw. die freien Basen selbst lassen sich entweder gar nicht oder nur schwer zurückgewinnen, sodaß eine erhebliche organische Verunreinigung des Abwassers auftritt und eine technische Realisierung des Verfahrens nahezu ausschließt.

In Anbetracht der Vielzahl von Nebenreaktionen und verfahrenstechnischer Probleme der bislang bekannten Herstellungsverfahren, besteht ein großes Bedürfnis nach einer verbesserten Synthesemöglichkeit zur Herstellung von hochreinen Fluoranilinen, wobei neben guten bis sehr guten Ausbeuten auch leicht zugängliche und im technischen Maßstab zur Verfügung stehende Vorstufen gefordert sind. Die reduktive Chlorabspaltung erweist sich als ein sehr günstiges Herstellungsverfahren, läßt sich aber aufgrund der hohen Korrosion, der ungünstigen Produktqualität und der nur schwer zu reproduzierbaren Versuchsergebnisse (Katalysator-Vergiftungen) bisher technisch kaum umsetzen. Es bestand daher ein großes Bedürfnis, die beschriebenen Mängel zu beseitigen und ein technisch günstiges Verfahren zu entwickeln.

Diese Aufgabe wird gelöst durch ein Verfahren zur Herstellung von Fluoranilinen der Formel (I)

FₙArNH₂ (I)

worin n 1, 2, 3 oder 4 ist, Ar für Phenyl, Naphthyl oder Pyridyl steht und die restlichen Substituenten am Ar-Rest gleich oder verschieden sind und unabhängig voneinander Wasserstoff, Halogen, (C₁-C₄)-Alkyl, Phenyl, NR₂, OR, CN, CHO, COR, wobei R Wasserstoff oder (C₁-C₆)-Alkyl ist, bedeuten, dadurch gekennzeichnet, daß man Fluornitrobenzole der Formel (II)

XₘFₙArNO₂ (II)

worin
n, Ar und die restlichen Substituenten am Ar-Rest die oben genannte Bedeutung besitzen, X für Chlor- oder Brom und m für 1, 2, 3 oder 4 steht, in Gegenwart eines Palladium-Katalysators, eines nicht wasserlöslichen Amins, das ebenfalls nicht wasserlösliche Hydrohalogenide bildet und gegebenenfalls eines inerten Lösungsmittels mit Wasserstoff umsetzt.

Die restlichen Substituenten sind alle Substituenten am Ar-Rest im Fluoranilin (I) außer Fₙ und NH₂ bzw. alle Substituenten am Ar-Rest im Fluornitrobenzol (II) außer Xₘ, Fₙ und NO₂. Sie stehen insbesondere für Wasserstoff, Halogen, (C₁-C₄)-Alkyl, OR, CN, COR, bevorzugt für Wasserstoff und Halogen.

Bei den Ausgangsverbindungen kann es sich um Brom oder Chlor-Verbindungen handeln, wie beispielsweise:
4-Chlor-2,3-difluornitrobenzol; 5-Chlor-2,3-difluornitrobenzol;
6-Chlor-2,3-difluornitrobenzol; 2-Chlor-3,4-difluornitrobenzol;
2-Chlor-4,5-difluornitrobenzol; 3-Chlor-4,5-difluornitrobenzol;
2-Chlor-5-fluornitrobenzol; 2,6-Dichlor-3,5-difluornitrobenzol;
3,5-Dichlor-2,6-difluornitrobenzol oder 3-Chlor-2,4-difluornitrobenzol.

In das Verfahren können auch Gemische von Verbindungen der Formel (II) eingesetzt werden, die nach Umsetzung eine einheitliche Verbindung der Formel (I) ergeben. Dabei können diese Verbindungen mit gleichem Substitutionsmuster bezüglich der Endverbindung auch als Mischungen verschiedener Chlor- und Bromverbindungen eingesetzt werden.

Besonders vorteilhaft lassen sich nach diesem Verfahren 3-Fluoranilin, 2,3-Difluoranilin, 3,4-Difluoranilin, 3,5-Difluoranilin und 2,6-Difluoranilin herstellen.

Es ist zweckmäßig den Katalysator auf einem Trägermaterial, wie beispielsweise Aktivkohle, Calciumcarbonat, Bariumsulfat, Bimsstein, Tonerde, Kieselgur, Kieselgel und/oder Aluminiumoxid anzuwenden. Bevorzugt wird Palladium auf Aktivkohle oder Aluminiumoxyd als Trägermaterial zur Anwendung gebracht.

Der Palladiumgehalt des Trägerkatalysators liegt bevorzugt bei 0,1 - 10 Gew.-%, vorzugsweise bei 0,2 bis 8 Gew.-% , besonders bevorzugt bei 0,5 - 6 Gew.-% Palladium, bezogen auf den gesamten Katalysator.

Die Menge des benötigten Katalysators liegt im Bereich von 0,1 - 50 mmol Palladium bezogen auf die Äquivalente abzuspaltendes Halogen (Chlor/Brom).

An Aminen können Monoamine oder Polyamine mit zwei bis vier Aminogruppen oder Gemische daraus dienen, mit der Eigenschaft, daß sowohl die freie Base als auch das mit der entstehenden HX gebildete Basenhydrohalogenid unter den Reaktions- und Aufarbeitungsbedingungen nicht wasserlöslich sind.

Sehr gute Ergebnisse werden erhalten, wenn sowohl die eingesetzten Amine als auch die entstehenden Hydrohalogenide flüssig sind.

Besonders geeignet sind Amine der allgemeinen Formel: (III)

HₚN(CᵣH₂ᵣ₊₁)_{q} (III)

wobei p = 0,1 oder 2; q = 1, 2 oder 3 und p + q = 3; r = 5 bis 20, bevorzugt 8 bis 15 und die Alkylreste gleich oder ungleich, verzweigt oder unverzweigt sein können. Insbesondere steht p für 0 oder 1 und q für 2 oder 3.

Hochwirksame aliphatische Amine seien im einzelnen Tri-(n-dodecyl)-amin; Tri-(iso-oktyl)-amin; Trialkyl-(C8/C10)-amine oder Mischungen aus ihnen.

Obwohl die vorstehend genannten Trialkylamine der genannten Formel (III) am geeignetsten sind, können prinzipiell auch Arylamine oder Aralkylamine eingesetzt werden.

Es hat sich in vielen Fällen bewährt mit Aminkonzentrationen von 50 bis 500 Mol% Amin pro Äquivalent abzuspaltendes Halogen zu arbeiten; bevorzugt wird das Amin in Mengen von 80 bis 250 Mol%, bevorzugt 100 bis 150 Mol% pro Äquivalent abzuspaltendes Halogen eingesetzt.

Bevorzugt wird in Gegenwart eines inerten Lösungsmittels in dem sowohl Edukte als auch Produkte gut löslich sind, gearbeitet. Die Reaktion kann auch ohne zusätzliches Lösungsmittel durchgeführt werden, falls es sich bei den verwendeten Edukten und Produkten um bei Reaktions- und Aufarbeitungstemperatur flüssige Verbindungen handelt. Als Lösungsmittel werden beispielsweise Benzol, Toluol, Xylol, Alkanole (C1-C4): Methanol, Ethanol Propanol, Polyglykole: Ethylenglykol, Dialkylether: Diethylether Methylethylether, Tetrahydrofuran, Pentan, Hexan, Heptan, Polyether: Polyethylenglykoldimethylether 500 oder Mischungen dieser Lösungsmittel verwendet.

Die Anwesenheit eines protischen Lösungsmittels (Wasser) ist in diesem Verfahren nicht erforderlich.

Das Verfahren kann sowohl bei Atmosphärendruck als auch bei Überdruck durchgeführt werden. Es ist zweckmäßig, bei einem Wasserstoffüberdruck von 0,1 bis 50 bar umzusetzen.

Es hat sich in vielen Fällen bewährt, das Verfahren bei Temperaturen von 0° bis 150°C, insbesondere 40° bis 120°C durchzuführen. Die Anwendung zu tiefer Temperaturen führt dabei zu einer langsamen und unvollständigen Reaktion. Zu hoch gewählte Temperaturen können zum Teil unerwünschte Fluorabspaltung oder Polymerbildung zur Folge haben.

Die Reduktion der Nitrogruppe und die reduktive Enthalogenierung können gleichzeitig durchgeführt werden, es ist jedoch auch möglich das Verfahren in zwei Schritten als Eintopfverfahren durchzuführen. Die Halogennitroverbindung wird hierbei in Gegenwart eines Katalysator und gegebenenfalls eines Lösungsmittels zuerst zum entsprechenden Halogenanilin reduziert, anschließend wird das oben beschriebene Amin zugegeben und die reduktive Enthalogenierung durchgeführt.

Das am Ende der Reaktion gebildete Aminhydrohalogenid kann auf einfache und vorteilhafte Weise regeneriert werden, indem die Rohlösung mit wässriger Base behandelt wird. Dabei bildet sich praktisch ohne Verluste das freie Amin, das in der Folgereaktion nach Abtrennen des Produktes, ohne weitere Vorbehandlung wieder eingesetzt werden kann.

Durch exakte Neutralisation des Aminhydrohalogenids wird nur soviel Base verbraucht, als Äquivalente Fluoraromat entstanden sind. Das entstehende Abwasser reagiert neutral.

Der bei der Reaktion anfallende gebrauchte Katalysator kann unbehandelt weiterverwendet, oder durch bekannte Reinigungsverfahren wie beispielsweise durch Wasserdampf gereinigt werden.

Die Ausgangsverbindungen des erfindungsgemäßen Verfahrens können durch Nitrierung der entsprechenden Chlorfluorbenzole bzw. durch Chlorfluoraustausch-Reaktionen an Chlornitroaromaten hergestellt werden. Die folgenden Beispiele dienen zur Erläuterung des erfindungsgemäßen Verfahrens ohne sich darauf zu beschränken.

### Beispiel 1

Zur Herstellung von 2,3 -Difluoranilin werden 96,8 g (0,5 mol) 3-Chlor-2,3-difluornitrobenzol, 2,0 g Pd/C (5%-ig, 50% wasserfeucht) als Katalysator, zusammen mit 300,0 g Toluol im Reaktionsgefäß (Autoklav) vorgelegt. Die Reaktionslösung wird auf 60°C aufgeheizt und bei dieser Temperatur mit Wasserstoff reduziert. Anschließend wird 295,5 g (0,74 mol) Tri-(C8/C10)alkylamin als Base zugegeben, die Temperatur auf 100°C erhöht und reduktiv entchloriert. Nach beendeter Wasserstoffaufnahme wird kurz nachgerührt, auf Raumtemperatur gekühlt, die Reaktionslösung mit Natronlauge neutralgestellt und der Katalysator vom Reaktionsgemisch abgenutscht. Nach dem Abtrennen der organische Phase wird diese im Vacuum andestilliert und das erhaltene Rohdestillat anschließend fraktioniert.

Die Aminmutterlauge sowie die aus der Fraktionierung resultierenden Produktionsrückstände (Vorläufe und Zwischenläufe) werden zurückgeführt.

| | | |
|---|---|---|
| Umsatz: | 100 % | (nach GC) |
| Ausbeute: | 58,4 g (0,45 mol) | 2,3-Difluoranilin |
| | 90,5 % | bezogen auf eingesetztes 4-Chlor-2,3-difluornitrobenzol. |
| Reinheit: | 98 (GC-Flächen-%) | 2,3-Difluoranilin |

### Beispiel 2

Zur Herstellung von 3,5-Difluoranilin werden 45,6 g (0,2 mol) 2,6-Dichlor-3,5-difluornitrobenzol, 2,5 g Pd/C (5%-ig, 50% wasserfeucht) als Katalysator, zusammen mit 100 g Toluol im Reaktionsgefäß (Autoklav) vorgelegt. Die Reaktionslösung wird auf 50°C aufgeheizt und bei dieser Temperatur mit Wasserstoff reduziert. Mit nachlassender Wasserstoffaufnahme wird 200 g (0,5 mol) Tri-(C8/C10)alkylamin als Base zudosiert, die Temperatur sukzessive auf 90°C erhöht und so lange bei dieser Temperatur gehalten, bis keine Wasserstoffaufnahme mehr zu beobachten ist. Es wird kurz nachgerührt, auf Raumtemperatur gekühlt, die Reaktionslösung mit Natronlauge neutralgestellt und der Katalysator vom Reaktionsgemisch abgenutscht. Nach dem Abtrennen der organische Phase wird diese bei reduziertem Druck andestilliert, das erhaltene Destillat getrocknet und anschließend fraktioniert.

Verbleibende Mutterlauge, Vorläufe und Zwischenläufe können in Folgeansätzen zurückgeführt werden.

| | | |
|---|---|---|
| Umsatz: | 99,0 % | (nach GC) |
| Ausbeute: | 23,7 g (0,18 mol) | 3,5-Difluoranilin |
| | 91,8 % | bezogen auf eingesetztes 2,6-Dichlor-3,5-difluornitrobenzol. |
| Reinheit: | 98 (GC-Flächen-%) | 3,5-Difluoranilin |

### Beispiel 3

Zur Herstellung von 3,4 -Difluoranilin werden 96,8 g (0,5 mol) 2-Chlor-3,4-difluornitrobenzol, 2,5 g Pd/C (5%-ig, 50% wasserfeucht) als Katalysator, zusammen mit 200,0 g Toluol und 4,8 g Tri-(C8/C10)alkylamin im Reaktionsgefäß (Autoklav) vorgelegt und bis zum Ende der Wasserstoffaufnahme reduziert. Anschließend werden 240,0 g (0,60 mol) Tri-(C8/C10)alkylamin als Base zugegeben und die Temperatur wird sukzessive von 60 auf 100°C erhöht und solange bei dieser Temperatur gehalten bis die Wasserstoffaufnahme erneut nachläßt. Nach beendeter Wasserstoffaufnahme wird kurz nachgerührt, auf Raumtemperatur gekühlt, die Reaktionslösung mit Natronlauge neutralgestellt und der Katalysator vom Reaktionsgemisch abgenutscht. Nach dem Abtrennen der organische Phase wird diese im Vacuum andestilliert das erhaltene Rohdestillat anschließend fraktioniert.

Die Aminmutterlauge sowie die aus der Fraktionierung resultierenden Produktionsrückstände (Vorläufe, Zwischenläufe) werden zurückgeführt.

| | | |
|---|---|---|
| Umsatz: | 100 % | (nach GC) |
| Ausbeute: | 55,4 g (0,43 mol) | 3,4-Difluoranilin |
| | 85,8 % | bezogen auf eingesetztes 2-Chlor-3,4-difluornitrobenzol. |
| Reinheit: | 98 (GC-Flächen-%) | 3,4-Difluoranilin |

## Patentansprüche

1. Verfahren zur Herstellung von Fluoranilinen der Formel (I)
FₙArNH₂ (I)
worin
n 1, 2, 3 oder 4 ist, Ar für Phenyl, Naphthyl oder Pyridyl steht und die restlichen Substituenten am Ar-Rest gleich oder verschieden sind und unabhängig voneinander Wasserstoff, Halogen, (C₁-C₄)-Alkyl, Phenyl, NR₂, OR, CN, CHO, COR, wobei R Wasserstoff oder (C₁-C₆)-Alkyl ist, bedeuten, dadurch gekennzeichnet, daß man Fluornitrobenzole der Formel (II)
XₘFₙArNO₂ (II)
worin
n, Ar und die restlichen Substituenten am Ar-Rest die oben genannte Bedeutung besitzen, X für Chlor- oder Brom und
m für 1, 2, 3 oder 4 steht, in Gegenwart eines Palladium-Katalysators, eines nicht wasserlöslichen Amins, das ebenfalls nicht wasserlösliche Hydrohalogenide bildet und gegebenenfalls eines inerten Lösungsmittels mit Wasserstoff umsetzt.

2. Verfahren nach Anspruch 1, dadurch gekennzeichnet, daß als geeignete Fluornitroaromaten der Formel (II)
4-Chlor-2,3-difluornitrobenzol, 5-Chlor-2,3-difluornitrobenzol,
6-Chlor-2,3-difluornitrobenzol, 2-Chlor-3,4-difluornitrobenzol,
2-Chlor-4,5-difluornitrobenzol, 3-Chlor-4,5-difluornitrobenzol,
2-Chlor-5-fluornitrobenzol, 2,6-Dichlor-3,5-difluornitrobenzol,
3,5-Dichlor-2,6-difluornitrobenzol oder 3-Chlor-2,4-difluornitrobenzol verwendet werden.

3. Verfahren nach mindestens einem der Ansprüche 1 bis 2, dadurch gekennzeichnet, daß Gemische von Verbindungen der Formel (II) eingesetzt werden, die nach Umsetzung eine einheitliche Verbindung der Formel (I) ergeben.

4. Verfahren nach mindestens einem der Ansprüche 1 bis 3, dadurch gekennzeichnet, daß 3-Fluoranilin; 3,4-Difluoranilin, 2,3-Difluoranilin, 2,5-Difluoranilin, 3,5-Difluoranilin oder 2,6-Difluoranilin hergestellt werden.

5. Verfahren nach mindestens einem der Ansprüche 1 bis 4, dadurch gekennzeichnet, daß bei Temperaturen von 0 bis 150°C, insbesondere 40° bis 120°C umgesetzt wird.

6. Verfahren nach mindestens einem der Ansprüche 1 bis 5, dadurch gekennzeichnet, daß als Palladium-Katalysator ein Palladium-Katalysator auf einem Trägermaterial eingesetzt wird.

7. Verfahren nach Anspruch 6, dadurch gekennzeichnet, daß als Trägermaterial Aktivkohle, Calciumcarbonat, Bariumsulfat, Bimsstein, Tonerde, Kieselgur, Kieselgel und/oder Aluminiumoxid eingesetzt wird.

8. Verfahren nach Anspruch 6 oder 7, dadurch gekennzeichnet, daß der Katalysator 0,1 - 10 Gew.-%, insbesondere 0,2 - 8 Gew.-%, bevorzugt 0,5 - 6 Gew.-% Palladium, bezogen auf das verwendete Trägermaterial, enthält.

9. Verfahren nach mindestens einem der Ansprüche 1 bis 8, dadurch gekennzeichnet, daß als Katalysator 0,01 bis 50 mmol Palladium, bezogen auf Äquivalente abzuspaltendes Halogen, eingesetzt werden.

10. Verfahren nach mindestens einem der Ansprüche 1 bis 9, dadurch gekennzeichnet, daß der Katalysator recyclisiert wird.

11. Verfahren nach mindestens einem der Ansprüche 1 bis 10, dadurch gekennzeichnet, daß als Amine Alkylamine eingesetzt werden.

12. Verfahren nach mindestens einem der Ansprüche 1 bis 11, dadurch gekennzeichnet, daß als Amin ein Amin der Formel (III)
HₚN(CᵣH₂ᵣ₊₁)_{q} (III)
wobei p = 0, 1 oder 2; q = 1, 2 oder 3 und p + q = 3; r = 5 bis 20, bevorzugt 8 bis 15 ist und die Alkylreste gleich oder ungleich, verzweigt oder unverzweigt sein können, eingesetzt wird.

13. Verfahren nach mindestens einem der Ansprüche 1 bis 12, dadurch gekennzeichnet, daß als Amine Tri-(n-dodecyl)-amin, Tri-(iso-oktyl)-amin, Trialkyl-(C8/C10)-amine oder Mischungen dieser Amine verwendet werden.

14. Verfahren nach mindestens einem der Ansprüche 1 bis 13, dadurch gekennzeichnet, daß die verwendeten Amine bei Reaktions- und Aufarbeitungstemperatur im Reaktionsmedium flüssig sind.

15. Verfahren nach mindestens einem der Ansprüche 1 bis 14, dadurch gekennzeichnet, daß die aus den verwendeten Aminen entstehenden Hydrohalogenide im Reaktionsmedium flüssig sind.

16. Verfahren nach mindestens einem der Ansprüche 1 bis 15, dadurch gekennzeichnet, daß das Alkylamin in Mengen von 50 bis 500 Mol%, insbesondere 80 bis 250 Mol%, bevorzugt 100 bis 150 Mol%, bezogen auf Äquivalente abzuspaltendes Halogen, verwendet wird.

17. Verfahren nach mindestens einem der Ansprüche 1 bis 16, dadurch gekennzeichnet, daß das Amin recyclisiert wird.

18. Verfahren nach mindestens einem der Ansprüche 1 bis 17, dadurch gekennzeichnet, daß bei Normal- oder Überdruck, insbesondere bei einem Wasserstoffüberdruck von 0,1 bis 50 bar umgesetzt wird.

19. Verfahren nach mindestens einem der Ansprüche 1 bis 18, dadurch gekennzeichnet, daß als Lösungsmittel Benzol, Toluol, Xylol, Alkanole (C1-C4): Methanol, Ethanol Propanol, Polyglykole: Ethylenglykol, Dialkylether: Diethylether Methylethylether, Tetrahydrofuran, Pentan, Hexan, Heptan, Polyether: Polyethylenglykoldimethylether 500 oder Mischungen dieser Lösungsmittel eingesetzt werden.

20. Verfahren nach mindestens einem der Ansprüche 1 bis 19, dadurch gekennzeichnet, daß die Halogennitroverbindung in Gegenwart des Katalysators und gegebenenfalls eines Lösungsmittels zum Halogenanilin reduziert und anschließend das Amin zugesetzt und die reduktive Enthalogenierung durchgeführt wird.

## Claims

1. A process for preparing fluoroanilines of the formula (I)
FₙArNH₂ (I)
in which
n is 1, 2, 3 or 4, Ar is phenyl, naphthyl or pyridyl, and the remaining substituents on the Ar radical are identical or different and are, independently of each other, hydrogen, halogen, (C₁-C₄)-alkyl, phenyl, NR₂, OR, CN, CHO or COR, where R is hydrogen or (C₁-C₆)-alkyl, which comprises reacting fluoronitrobenzenes of the formula (II)
XₘFₙArNO₂ (II)
in which
n, Ar and the remaining substituents on the Ar radical have the abovementioned meaning, X is chlorine or bromine and
m is 1, 2, 3 or 4, with hydrogen in the presence of a palladium catalyst, of an amine which is not soluble in water and which also does not form watersoluble hydrohalides, and, where appropriate, of an inert solvent.

2. The process as claimed in claim 1, wherein 4-chloro-2,3-difluoronitrobenzene, 5-chloro-2,3-difluoronitrobenzene, 6-chloro-2,3-difluoronitrobenzene, 2-chloro-3,4-difluoronitrobenzene, 2 chloro-4,5-difluoronitrobenzene, 3-chloro-4,5-difluoronitrobenzene, 2-chloro-5-fluoronitrobenzene, 2,6-dichloro-3,5-difluoronitrobenzene, 3,5-dichloro-2,6-difluoronitrobenzene or 3-chloro-2,4-difluoronitrobenzene
are used as suitable fluoronitro aromatic compounds of the formula (II).

3. The process as claimed in at least one of claims 1 to 2, wherein mixtures of compounds of the formula (II) are employed which, after reaction, yield a homogeneous compound of the formula (I).

4. The process as claimed in at least one of claims 1 to 3, wherein 3-fluoroaniline, 3,4-difluoroaniline, 2,3-difluoroaniline, 2,5-difluoroaniline, 3,5-difluoroaniline or 2,6-difluoroaniline are prepared.

5. The process as claimed in at least one of claims 1 to 4, wherein reaction takes place at temperatures of from 0 to 150°C, in particular of from 40° to 120°C.

6. The process as claimed in at least one of claims 1 to 5, wherein a palladium catalyst on a support material is employed as the palladium catalyst.

7. The process as claimed in claim 6, wherein active charcoal, calcium carbonate, barium sulfate, pumice stone, alumina, kieselguhr, silica gel and/or aluminum oxide is employed as support material.

8. The process as claimed in claim 6 or 7, wherein the catalyst contains 0.1 - 10% by weight, in particular 0.2 - 8% by weight, preferably 0.5 - 6% by weight, of palladium, based on the support material used.

9. The process as claimed in at least one of claims 1 to 8, wherein from 0.01 to 50 mmol of palladium, based on equivalents of halogen to be eliminated, are employed as catalyst.

10. The process as claimed in at least one of claims 1 to 9, wherein the catalyst is recycled.

11. The process as claimed in at least one of claims 1 to 10, wherein alkylamines are employed as amines.

12. The process as claimed in at least one of claims 1 to 11, wherein an amine of the formula (III)
HₚN(CᵣH₂ᵣ₊₁)_{q} (III)
where p = 0, 1 or 2; q = 1, 2 or 3 and p + q = 3; r = from 5 to 20, preferably from 8 to 15, and the alkyl radicals may be identical or different and branched or unbranched, is employed as the amine.

13. The process as claimed in at least one of claims 1 to 12, wherein tri(n-dodecyl)amine, tri(isooctyl)amine or trialkyl(C8/C10)amines, or mixtures of these amines, are used as amines.

14. The process as claimed in at least one of claims 1 to 13, wherein the amines which are used are liquid in the reaction medium at the reaction temperature and the working-up temperature.

15. The process as claimed in at least one of claims 1 to 14, wherein the hydrohalides resulting from the amines which are used are liquid in the reaction medium.

16. The process as claimed in at least one of claims 1 to 15, wherein the alkylamine is used in quantities of from 50 to 500 mol%, in particular of from 80 to 250 mol%, preferably of from 100 to 150 mol%, based on equivalents of halogen to be eliminated.

17. The process as claimed in at least one of claims 1 to 16, wherein the amine is recycled.

18. The process as claimed in at least one of claims 1 to 17, wherein reaction takes place under standard pressure or excess pressure, in particular under a hydrogen excess pressure of from 0.1 to 50 bar.

19. The process as claimed in at least one of claims 1 to 18, wherein benzene, toluene, xylene, alkanols (C1-C4): methanol, ethanol, propanol, polyglycols: ethylene glycol, dialkyl ethers: diethyl ether, methyl ethyl ether, tetrahydrofuran, pentane, hexane, heptane, polyethers: polyethylene glycol dimethyl ether 500, or mixtures of these solvents, are employed as solvents.

20. The process as claimed in at least one of claims 1 to 19, wherein the halonitro compound is reduced to the haloaniline in the presence of the catalyst and, where appropriate, of a solvent, and the amine is then added and the reductive dehalogenation is carried out.

## Revendications

1. Procédé de préparation de fluoranilines de formule (I)
FₙArNH₂ (I)
dans laquelle n vaut 1, 2, 3 ou 4, Ar représente phényle, naphtyle ou pyridyle et les substituants restants sont identiques ou différents sur le reste Ar et, indépendamment l'un de l'autre, représentent l'hydrogène, halogène, alkyle en C₁-C₄, phényle, NR₂, OR, CN, CHO, COR, R étant l'hydrogène ou alkyle en C₁-C₆, caractérisé en ce que l'on fait réagir des fluoronitrobenzènes de formule(II)
XₘFₙArNO₂ (II)
dans laquelle
n, Ar et les autres substituants sur le reste Ar ont les significations données ci-dessus, X représente le chlore ou le brome et m vaut 1, 2, 3 ou 4, en présence d'un catalyseur au palladium, d'une amine non hydrosoluble, qui ne forme pas non plus des halogénures d'hydrogène hydrosolubles et éventuellement dans un solvant inerte, avec l'hydrogène.

2. Procédé selon la revendication 1, caractérisé en ce que l'on utilise en tant que fluoronitroaromatiques appropriés de formule II :
le 4-chloro-2,3-difluoronitrobenzène ;
le 5-chloro-2,3-difluoronitrobenzène ;
le 6-chloro-2,3-difluoronitrobenzène ;
le 2-chloro-3,4-difluoronitrobenzène ;
le 2-chloro-4,5-difluoronitrobenzène ;
le 3-chloro-4,5-difluoronitrobenzène ;
le 2-chloro-5-fluoronitrobenzène ;
le 2,6-dichloro-3,5-difluoronitrobenzène ;
le 3,5-dichloro-2,6-difluoronitrobenzène ; ou
le 3-chloro-2,4-difluoronitrobenzène.

3. Procédé selon au moins l'une des revendications 1 à 2, caractérisé en ce que l'on utilise des mélanges de composés de formule (II), qui donnent après réaction un composé homogène de formule (I).

4. Procédé selon au moins l'une des revendications 1 à 3, caractérisé en ce que l'on prépare la 3-fluoraniline, la 3,4-difluoraniline, la 2,3-difluoraniline, la 2,5-difluoraniline, la 3,5-difluoraniline ou la 2,6-difluoraniline.

5. Procédé selon au moins l'une des revendications 1 à 4, caractérisé en ce que l'on effectue la réaction à des températures de 0 à 150 °C, plus particulièrement de 40 à 120 °C.

6. Procédé selon au moins l'une des revendications 1 à 5, caractérisé en ce que l'on utilise en tant que catalyseur au palladium un catalyseur au palladium sur une matière de support.

7. Procédé selon la revendication 6, caractérisé en ce que l'on utilise en tant que matière de support le charbon actif, le carbonate de calcium, le sulfate de baryum, la pierre ponce, l'argile, le kieselgur, le gel de silice et/ou l'oxyde d'aluminium.

8. Procédé selon la revendication 6 ou 7, caractérisé en ce que le catalyseur contient de 0,1 à 10 % en poids, plus particulièrement de 0,2 à 8 % en poids, de préférence de 0,5 à 6 % en poids de palladium par rapport à la matière de support utilisée.

9. Procédé selon au moins l'une des revendications 1 à 8, caractérisé en ce que l'on utilise en tant que catalyseur de 0,01 à 50 mmoles de palladium par rapport aux équivalents d'halogène à dissocier.

10. Procédé selon au moins l'une des revendications 1 à 9, caractérisé en ce que l'on recycle le catalyseur.

11. Procédé selon au moins l'une des revendications 1 à 10, caractérisé en ce que l'on utilise en tant qu'amines des alkylamines.

12. Procédé selon au moins l'une des revendications 1 à 11, caractérisé en ce que l'on utilise en tant qu'amines une amine de formule (III) :
HₚN(CᵣH₂ᵣ₊₁)_{q} (III)
dans laquelle p = 0, 1 ou 2 ; q = 1, 2 ou 3 et p + q = 3 ; r = 5 à 20, de préférence de 8 à 15 et les restes alkyle peuvent être identiques ou différents, ramifiés ou linéaires.

13. Procédé selon au moins l'une des revendications 1 à 12, caractérisé en ce que l'on utilise en tant qu'amines la tri-(n-dodécyl)amine, la tri-(iso-octyl)amine et la tri-(alkyl en C₈-C₁₀)-amine, ou les mélanges de ces amines.

14. Procédé selon au moins l'une des revendications 1 à 13, caractérisé en ce que les amines utilisées à des températures réactionnelles et de traitement sont liquides dans le milieu réactionnel.

15. Procédé selon au moins l'une des revendications 1 à 14, caractérisé en ce que les halogénures d'hydrogène formés à partir des amines utilisées sont liquides dans le milieu réactionnel.

16. Procédé selon au moins l'une des revendications 1 à 15, caractérisé en ce que l'on utilise l'alkylamine à raison de 50 à 500 % en moles, plus particulièrement de 80 à 250 % en moles, de préférence de 100 à 150 % en moles par rapport aux équivalents d'halogènes à dissocier.

17. Procédé selon au moins l'une des revendications 1 à 16, caractérisé en ce que l'on recycle l'amine.

18. Procédé selon au moins l'une des revendications 1 à 17, caractérisé en ce que l'on met en oeuvre la réaction à une pression normale ou sous une surpression, plus particulièrement à une surpression d'hydrogène de 0,1 à 50 bars.

19. Procédé selon au moins l'une des revendications 1 à 18, caractérisé en ce que l'on utilise comme solvants le benzène, le toluène, le xylène, des alcanols en C₁-C₄ : le méthanol, l'éthanol, le propanol, le polyglycol, l'éthylèneglycol, le dialkyléther, le diéthyléther, le méthyléthyléther, le tétrahydrofuranne, le pentane, l'hexane, l'heptane, des polyéthers, le polyéthylèneglycoldiméthyléther 500, ou les mélanges de ces solvants.

20. Procédé selon au moins l'une des revendications 1 à 19, caractérisé en ce que l'on réduit le composé halogénonitré en présence du catalyseur et éventuellement d'un solvant en l'halogénoaniline et ensuite on ajoute l'amine et on met en oeuvre la déshalogénation réductrice.
